# EUROPEAN PATENT APPLICATION

(11) **EP 1 974 722 A2**
(43) Date of publication of application: **01.10.2008**
(21) Application number: 08006027.0
(22) Date of filing: 28.03.2008
(51) Int. Cl.: A61K 9/14, A61K 9/16, A61K 9/50, A61K 31/436

(54) **Modified dosage forms of tacrolimus**

(30) Priority: 29.03.2007 IN MU05832007
(71) Applicant: Panacea Biotec Ltd, Mumbai 400099 (IN)
(72) Inventor: Jain, Rajesh, New Dehli - 110 044 (IN); Singh, Amarjit, Andheri (East) Mumbai - 400 099, Maharashtra (IN); Singh, Sarabjit, Andheri (East) Mumbai - 400 099, Maharashtra (IN); Puthli, Shivanand, Andheri (East) Mumbai - 400 099, Maharashtra (IN)
(74) Representative: Westphal, Thomas

(57) **Abstract**

The present invention provides a modified release dosage form of tacrolimus that releases two or more amount of tacrolimus upon oral administration, the first amount of tacrolimus releases from the immediate release dosage unit substantially immediately within 0-2 hours followed by a time interval ranging from about 1-10 hours during which substantially no amount of tacrolimus is released from the dosage form, after which a second amount of tacrolimus is released wherein said second amount is released from the delayed release dosage unit either immediately e.g. within 0-2 hours or over a period of time ranging from about 2-12 hours from its initial release from the delayed release dosage unit. The dosage form may further comprise additional amount of tacrolimus to provide additional pulse of tacrolimus. The dosage forms of tacrolimus exhibit improved bioavailability and reduced flux or fluctuation over existing composition of tacrolimus. A method of preparing the dosage forms is also described.

## Description

### FIELD OF THE INVENTION

The present invention relates to modified release dosage forms of tacrolimus which exhibits improved bioavailability and reduced pharmacokinetic inter-individual variability.

### BACKGROUND OF THE INVENTION

Tacrolimus, known as FK-506 is a macrolide immunosuppressant produced by *Streptomyces tsukubaensis.* Tacrolimus is available in various dosage forms such as capsules, injections and an ointment. The conventional capsule dosage form is sold commercially as Prograf^{®} and is approved for prophylaxis of organ rejection in patients receiving allogeneic liver, kidney or heart transplants. Absorption of orally administered tacrolimus from the gastrointestinal tract is incomplete and variable. The absolute bioavailability of tacrolimus is typically 17±10% in adult kidney transplant patients (N=26), typically 22±6% in adult liver transplant patients (N=17), typically 23±9% in adult heart transplantation patients (N=11) and typically 18±5% in healthy volunteers (N=16). It has been observed that the absorption is affected by the presence of food. The rate and extent of tacrolimus absorption is greatest under fasted conditions. The presence and composition of food decreases both the rate and extent of tacrolimus absorption. Prograf^{®} is available in doses of 0.5mg, 1mg or 5mg and is administered in two divided daily doses every 12 hours.

Tacrolimus pharmacokinetic characteristics vary greatly among individuals. It is subject to substantial intestinal and hepatic first-pass effects. Tacrolimus is a substrate of cytochrome p450 (CYP3A), it has poor bioavailability because of this extensive metabolism and its bioavailability is individually variable. Thus, tacrolimus elimination, expressed as total body clearance, varies interindividually from 0.041 to 0.36 L · h⁻¹ · (kg of body weight)⁻¹. Because of this variability in conjunction with its narrow therapeutic index, monitoring of whole-blood concentrations of tacrolimus is essential to achieve optimal efficacy while minimizing the risk of toxicity.

A prolonged release once-a-day oral capsule dosage form available in doses of 0.5mg, 1mg and 5mg is sold commercially as Advagraf^{®} and is indicated for prophylaxis of transplant rejection in adult kidney, liver or heart allograft recipients. The label states that the therapy requires careful monitoring by adequately qualified and equipped personnel; its dosing should primarily be based on clinical assessments of rejections and tolerability in each patient individually, aided by blood level monitoring. Following conversion of patients from Prograf^{®} to Advagraf^{®}, monitoring of tacrolimus trough levels is still required and when necessary dose adjustments are made to maintain similar systemic exposure, keeping in mind that it may take several days after starting the Advagraf^{®} dose regimen to achieve steady state. In stable patients converted from Prograf^{®} (twice daily) to Advagraf^{®} (once-daily) on a 1 : 1 (mg : mg) total daily dose basis, the systemic exposure to tacrolimus (AUC₀₋₂₄) for Advagraf^{®} was approximately 10% lower than that for Prograf^{®}.

United States Patent 6,440,458, United States Patent 6,576,259 and United States Patent 6,884,433 relate to sustained release formulations comprising a macrolide, tacrolimus, wherein the time required for 63.2% (T63.2%) of the maximum amount of tacrolimus or its hydrate to be dissolved is 0.7 to 15 hours, as measured according to Japanese Pharmacopoeia 13^{th} edition Dissolution Test no. 2 (Paddle method, 50 rpm) in a test solution which is aqueous 0.005% hydroxypropyl cellulose, adjusted to pH 4.5.

United States Application Publication 2006/0287352 relates to a modified release composition comprising tacrolimus which releases less than 20% w/w of the active ingredient within 0.5 hrs when subjected to an in-vitro dissolution test using USP paddle method and using 0.1N HCl as dissolution medium. The modified release composition may be enteric-coated and/or may comprise a solid dispersion or solid solution of tacrolimus in a hydrophilic or water-soluble vehicle and one or more modifying release agents; and/or may comprise a solid dispersion or solid solution of tacrolimus in an amphiphilic or hydrophobic vehicle and optionally one or more modifying release agents. The modified release dosage forms of tacrolimus of the '352 application mentioned above delays the release of tacrolimus to the distal part of duodenum thus reducing drug related gastrointestinal side-effects and the relatively high degree of metabolism in the proximal part of the gastrointestinal tract (CYP3A4 mediated metabolism), however the therapeutic utility is limited by the failure to provide any rapid onset.
United States Patent 6,569,463 describes a pharmaceutical composition in the form of solid carrier comprising a sub-strate and encapsulation coat wherein the encapsulation coat comprises an admixture of a therapeutically effective amount of hydrophobic pharmaceutical active ingredient, an effective solubilizing amount of at least one hydrophilic surfactant and lipophilic additive selected from the group consisting of lipophilic surfactants, triglycerides, and combinations thereof, wherein the effective solubilizing amount of the at least one hydrophilic surfactant is an amount effective to partially or fully solubilize the pharmaceutical active ingredient in the encapsulation coat. According to the patent, the solid carrier for pharmaceutical active ingredients offer potential advantages over known micronized drugs, emulsion or solubilized formulation in enhancing in-vivo performance of hydrophobic drugs.

As mentioned above, the various concerns with tacrolimus therapy are the extensive metabolism, incomplete and variable absorption, low bioavailability, food effect and side-effects. Monitoring and maintaining tacrolimus whole blood concentrations in patients after oral administration is essential to avoid rejection, toxicity and to improve compliance as increasing tacrolimus trough blood concentrations increases incidence of adverse events. Improving compliance is important because of the probability of noncompliance in any lifelong therapeutic regimen.

There is definitely a need to provide a more effective and compliant therapy for this immunosuppressant in organ transplantation.

### SUMMARY OF THE INVENTION

The invention relates to modified release dosage forms of tacrolimus, which addresses most of the issues with tacrolimus therapy by providing dosage forms which release tacrolimus in such a manner that it eliminates the release of tacrolimus when a dosage form passes through the metabolizing region of cytochrome p450 system (CYP3A). The modified release dosage form of tacrolimus comprises an immediate release component and at least one modified release component, which releases the drug in such a manner that it exhibits reduced metabolism and improved bioavailability. Improved bioavailability may allow reduction in dose for treatment and may also reduce or eliminate the food effect, resulting in increased patient compliance. Further the peak to trough ratio (flux) is significantly reduced to provide a less variable release profile in comparison to the commercially available products now on the market.

### BRIEF DESCRIPTION OF THE DRAWINGS

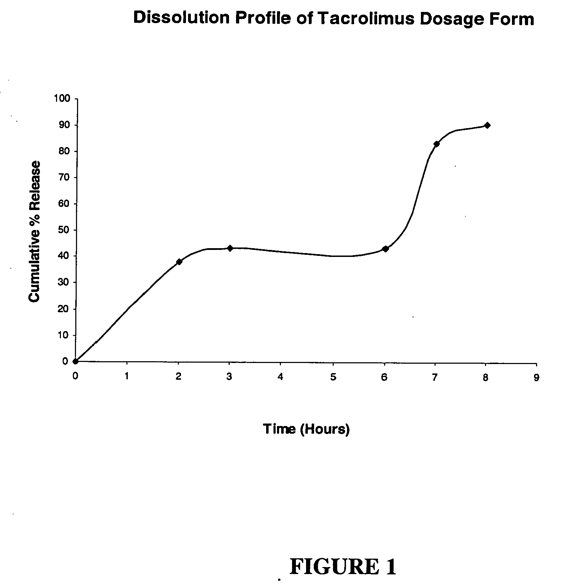

Figure 1 illustrates the dissolution profile of composition E described in Table 1 of the present invention.

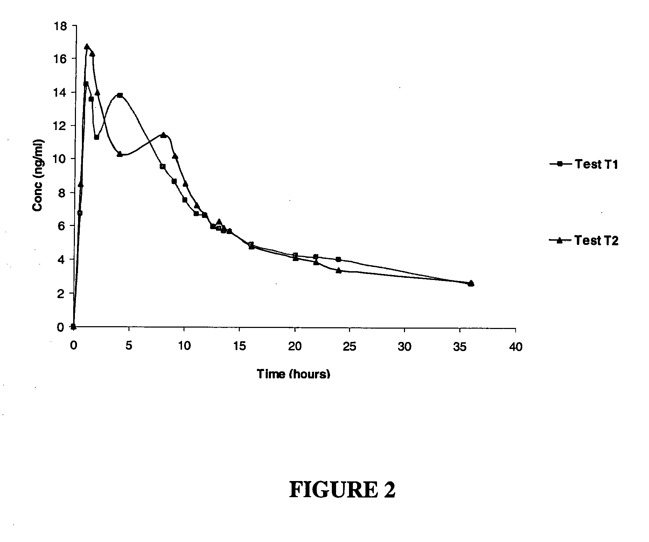

Figure 2 illustrates in-vivo data of compositions E and J (Test T₁ and T₂) described in Table 1 and Table 2 respectively.

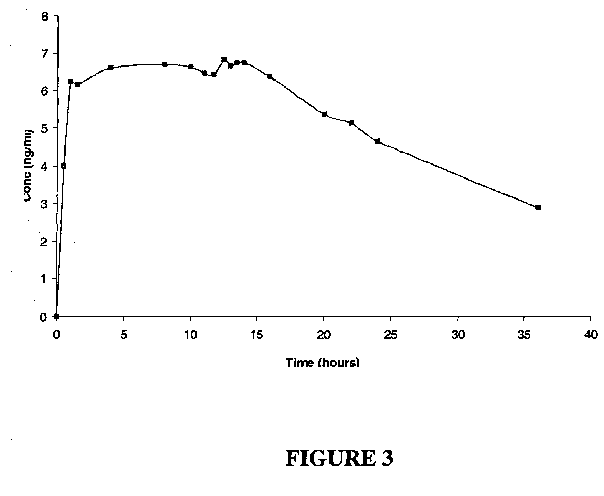

Figure 3 illustrates in-vivo data of composition K described in Table 2.

### DETAILED DESCRIPTION OF THE INVENTION

Various aspects of the invention provide -
1. A dosage form of tacrolimus which due to its formulation characteristics avoids cytochrome P450 metabolism and at the same time provides an initial drug pulse to achieve the fast onset of action and at a later time provides pulses to provide peak plasma concentration and to maintain a trough concentration to reduce the risk of rejection of the transplanted organ.
2. A dosage form of tacrolimus which in operation releases a first amount of tacrolimus immediately and the second amount of tacrolimus after a pre-determined time interval.
3. A dosage form of tacrolimus capable of releasing the drug in bimodal or multi-modal manner in which first amount of tacrolimus is released immediately and one or more additional amounts of tacrolimus are released each after a respective time interval to provide additional pulses of drug release.
4. A modified release once-daily dosage form of tacrolimus having reduced metabolism and improved bioavailability.
5. A modified release dosage form of tacrolimus having reduced flux or fluctuation.
6. A modified release dosage form of tacrolimus having C₂₄ levels equivalent or similar to that achieved by administration of two immediate release dosage forms given sequentially.

The above aspects are realized by a modified release dosage form of tacrolimus that releases two or more amounts of tacrolimus upon oral administration wherein the first amount of tacrolimus is released substantially immediately followed by a time interval during which substantially no amount of tacrolimus is released from the dosage form, after which the second amount of tacrolimus is released. Additional amounts are released subsequently after additional time intervals.

Accordingly the modified release dosage form of tacrolimus contains at least two dosage units, one dosage unit is the immediate release dosage unit and the second dosage unit is the delayed release dosage unit such that the first amount of tacrolimus is present in the immediate release dosage unit and the second amount of tacrolimus is present in the delayed release dosage unit. The first amount of tacrolimus is present in an amount of about 10% w/w to about 70% w/w of the total amount of tacrolimus and the second amount of tacrolimus is present in an amount of about 30% w/w to about 90% w/w of the total amount of tacrolimus in the dosage form. The dosage form may contain more than one delayed release dosage units.

In one aspect, a modified release dosage form of tacrolimus of the present invention releases the first amount of tacrolimus from the immediate release dosage unit substantially immediately within 0-2 hours followed by a time interval ranging from about 1-10 hours during which substantially no amount of tacrolimus is released from the dosage form, after which a second amount of tacrolimus is released wherein said second amount is released from the delayed release dosage unit either immediately e.g. within 0-2 hours or over a period of time ranging from about 2-12 hours from its initial release from the delayed release dosage unit.

In another aspect of the invention, a modified release dosage form of tacrolimus exhibits at least one of (a) an in-vitro release profile wherein 0- 50% of the total drug is released in 2.0 hrs; after 4 hrs 20-60% of drug is released and not less than 70% of total drug is released after 8 hrs when tested by pH change method and (b) an in-vivo profile wherein C₄ is within the range from about 6 ng/ml to 18 ng/ml and/or C₂₄ is within the range from about 3 ng/ml to 20 ng/ml following single dose oral administration of 5mg tacrolimus.

In yet another aspect of the invention, a modified release dosage form of tacrolimus exhibits at least one of (a) an in-vitro release profile wherein 0- 50% of the total drug is released in 2.0 hrs; after 4 hrs 20-60% of drug is released and not less than 70% of total drug is released after 8 hrs when tested by pH change method and (b) an in-vivo plasma profile wherein the peak-to-trough ratio after a single dose oral administration ranges from about 6.5 to 1.5, preferably it ranges from about 3.0 to 1.5.

In yet another aspect of the invention, a modified release dosage form of tacrolimus exhibits at least one of (a) an in-vitro release profile wherein 0- 50% of the total drug is released in 2.0 hrs; after 4 hrs 20-60% of drug is released and not less than 70% of total drug is released after 8 hrs when tested by pH change method and (b) an in-vivo plasma profile wherein the extrapolated steady state fluctuation (Flux) is reduced by about 40% to 70% of the commercially available products described herein indicating reduced pharmacokinetic variability.

In another aspect of the invention, the modified release dosage form of tacrolimus comprises (i) immediate release dosage unit and (ii) one or more delayed release dosage units wherein said dosage form when tested in a USP Type II apparatus at 75 rpm using a pH change method exhibits a dissolution profile substantially corresponding to: more than 20% of the tacrolimus is released in 0.5 hours; after 4 hours 20-60% of the tacrolimus is released; and after 8 hours not less than 70% of the tacrolimus is released.

It is to be understood that this invention is not limited to the particular systems, process steps, and materials disclosed herein as modification to these may occur to a person skilled in the art. It is also to be understood that the terminology employed herein is used for the purpose of describing particular aspects only and is not intended to be limiting since the scope of the present invention will be limited only by the appended claims and equivalents thereof.

It must be noted that as used in the specification and the appended claims, the singular forms 'a', 'an' and 'the' include plural references unless the context clearly indicates otherwise. Thus for example, use of the term 'an active agent' includes reference to one or more active agents.

As disclosed herein and as used in the compositions and methods of the present invention, the term 'tacrolimus', includes tacrolimus, any of its pharmaceutically acceptable salts, and any of its conjugates, derivatives, complexes, prodrugs and natural and synthetic analogues, solvate, hydrate or anhydrates thereof. Use of the term 'drug' or 'active ingredient' in context of the present invention refers to tacrolimus including the forms mentioned herewith. Amorphous form, crystals, polymorphs or any other forms of tacrolimus are also included herewith.

The term "modified release" as used herein in relation to the composition according to the invention or a coating material or used in any context means release which is not immediate release and is taken to encompass controlled release, sustained release, timed release, retarded release, extended release, programmed release, pulsatile release, burst release and delayed release.

The term "immediately" or "substantially immediately" as used herein refers to release of drug within 0 to 2 hours of administration, "pre-determined time interval" as used herein refers to the time between delivery of drug from one dosage unit and the subsequent delivery of drug from another dosage unit.

The present invention provides a modified release dosage form of tacrolimus comprising at least two dosage units; at least one immediate release dosage unit and at least one delayed release dosage unit, such that the composition provides a pulsatile release of tacrolimus. As used herein, term "pulsatile release" means any pulsatile release system that releases the drug in "pulses," wherein a single dosage form provides a first amount of tacrolimus substantially immediately upon administration to a mammal followed by a predetermined time interval of "substantially no release" after which a second amount of tacrolimus is released either immediately or over a period of time. The dosage form provides enhanced bioavailability in mammals, significantly reduced metabolism, reduced pharmacokinetic variability and provides a patient compliant once-a-day therapy of tacrolimus. In certain aspects, the modified release dosage form may comprise one immediate release dosage unit and two or more delayed release dosage units, delivering the drug in two or more pulses. The term "dosage units", as described herein encompasses beads, granules, pellets, particles or mini-tablets filled into capsules or sachets or compressed into tablets, each dosage unit releasing amounts of tacrolimus as pulses, at different time interval(s) encompassing a predetermined time interval of "substantially no release", each amount being substantially the same or different and providing the total recommended daily dose. The term 'substantially no release' is defined as release of the drug from the dosage form which ranges from about 0% to about 10% of the total dose. The term 'dosage form' (or interchangeably termed as 'composition') encompasses the final form in which the drug is administered to the mammal, which includes apart from others tablet, capsule and sachets. Mammal used in context of this invention refers to 'human being'.

Dissolution by 'pH change method' as used in this context means carrying out the dissolution in pH 1.2 for 2 hrs, followed by pH 4.5 for 1 hr, pH 6.8 for 1 to 3 hrs and further dissolution in pH 7.4.

The term 'solid dispersion' as used herein refers to drug or active ingredient dispersed or adsorbed in a carrier and/or inert material, usually as fine particulate dispersion. Carrier or Inert materials are selected from beads, non-pareil seeds, granules, pellets, particles, and powder and core tablets.

The term 'about' with respect to the composition can mean plus or minus a range of up to 20%, preferably up to 10%, more preferably up to 5%. This term particularly with respect to biological systems or processes can mean within an order of magnitude, preferably within 5-fold, and more preferably within 2-fold, of a value. Where particular values are described in the application and claims, unless otherwise stated the term "about" means within an acceptable error range for the particular value as determined by one of ordinary skill in the art.

The term 'bioavailability' denotes the rate and extent to which a drug is available at the site of action after administration, 'Cₘₐₓ' as used herein means maximum plasma concentration of tacrolimus achieved on administration of the dosage form to a mammal (it can be considered as being synonymous to 'peak levels'). 'Cₘᵢₙ' as used herein means minimum plasma concentration of tacrolimus achieved on administration of the dosage form to a mammal (it can be considered as being synonymous to 'trough levels'). 'C₂₄' levels means plasma concentration at 24 hours after administration of dosage form, which at times is same as the Cₘᵢₙ. "Tₘₐₓ' as used herein means the time to achieve maximum plasma concentration after administration of the dosage form. 'T₁' and 'T₂' when used indicates the time to achieve maximum concentration of first peak and time to achieve maximum concentration of second peak on administration of the dosage form, wherein the two peaks are distinct. 'Steady-State Fluctuation' or 'Flux' as used herein is expressed as : Flux = [Cₘₐₓ-Cₘᵢₙ)/C_{avg}] * 100%; where C_{avg} = AUC₀₋/ .. wherein is 24 hrs. A reduced % Flux indicates a reduced pharmacokinetic variability. 'Peak-to-trough ratio' is = Cₘₐₓ/C₂₄.

Commercially available immediate release product or immediate release product as described herein is Prograf^{®} and commercially available prolonged release product or prolonged release product as described herein is Advagraf^{®}.

Therapeutic drug monitoring especially in immunosuppressant therapy plays an important role in predicting efficacy and safety parameters of the drug. Although drug monitoring is not required for all the drugs, it is necessary for those drugs which meet the following characteristics: narrow therapeutic window, high inter-patient and/or intra-patient pharmacokinetic variability and significant risk of noncompliance. Tacrolimus is one such drug which meets most of all the above characteristics. Researchers have found that the whole blood trough concentration (C₂₄ or Cₘᵢₙ) of tacrolimus plays an important role in evaluating the efficacy of tacrolimus therapy. Blood concentration at C₄ and/or Cₘₐₓ determines the toxicity level of tacrolimus.

Keeping this in mind a modified release dosage form of tacrolimus has been developed that provides a once-a-day therapy with a substantially similar C₂₄ level to that obtained by administering two or more immediate release doses of tacrolimus sequentially and the C₂₄ levels is at least about 1.3 times; at least about 1.5 times; at least about 1.8 times that of a commercially available prolonged release product. In certain aspects, the C₂₄ value ranges from about 3.0 ng/ml to about 20 ng/ml preferably 3.0 ng/ml to about 10 ng/ml. The dosage form also exhibits an AUC_{0-inf} /AUC_{0-inf} of a commercially available immediate release product value of at least about 0.9 to about 3.0 or more; preferably about 1.0 to about 2.0 or more, the AUC values being a ratio of the values for the dosage form of present invention to the value obtained for the commercially available immediate release product described herein, determined under similar conditions. The peak-to-trough ratio which determines the variability and safety of the product is about 3.5 to 4.5 in the commercially available immediate release product described herein, is about 3.5 to 5.5 in the commercially available prolonged release product and is about 1.5 to 3.0 for the preferred compositions of the present invention indicating reduced variability in the compositions of the present invention. In certain compositions the peak-to-trough ratios were found to be similar.

Another parameter demonstrating pharmacokinetic variability is the steady state fluctuation or % flux values, wherein a reduced flux indicates reduced variability, the values for which for the modified release dosage form of the present invention is reduced by about 40% to 70% of the commercially available products described herewith.

Furthermore, a modified release dosage form according to the invention described herewith when administered orally provides an extended period of time during which the plasma concentration is maintained within the therapeutic window leading to reduced side-effects. In certain aspects a constant plasma concentration or a plasma concentration which is between at least 30% or more of the Cₘₐₓ and 70% or more of the Cₘₐₓ is maintained over a period of at least about 2-20 hours, preferably at least about 4-10 hours. The dosage form exhibits a Cₘₐₓ that is reduced, about 80% of that of the Cₘₐₓ of the commercially available immediate release product (Prograf^{®}), preferably about 70%, more preferably about 50% or still more preferably about 40%. The Cₘₐₓ value being at the most about 40 ng/ml, or at the most about 20 ng/ml, or at the most about 10 ng/ml.

In certain aspects the dosage form may contain from 0.5 mg to 10 mg of tacrolimus per dosage form. In an example of the invention, the dosage form contains 5 mg of tacrolimus.

The dosage form according to this invention will also provide a Coefficient of Variation (CV) on Area Under Curve data that is significantly lower than the commercially available immediate release product and lower than the commercially available prolonged release product described herein.

As encompassed by the invention, the release of an amount of tacrolimus from each dosage unit may be rapid (i.e. 'rapid release' up to 2 hours) or the release may be extended over a period of time (i.e. 'extended release' over more than about 2 hours to about 12 hours from its time of release) Thus the dosage form may be manufactured in a combination of rapid release and/or extended release amounts. For example, it may be a combination of two rapid release dosage units, or a combination of three rapid release dosage units, or a combination of first rapid release and second extended release dosage units, or a combination of first extended release and second rapid release dosage units, or a combination of first rapid release and two or more extended release dosage units, combination of two or more extended release dosage units and so on. On administration, the dosage form releases tacrolimus amounts as per the desired pulsatile profile. On absorption, this may lead to any type of pulsatile or continuous profile in vivo, depending upon the formulation.

A pulsatile release profile would significantly reduce or avoid the CYP3A4 metabolism of tacrolimus, enhancing its bioavailability, reducing the effective dose and reducing side-effects. Increased bioavailability, AUC (area under the curve), would reduce the intra- and inter-variability related to absorption of the drug, thus providing a reduced coefficient of variation or reduced pharmacokinetic inter-individual variability.

It is to be understood that, the dosage form when administered orally, once daily, it releases the drug as two or more pulses or peaks, wherein in certain aspects, when there is a sufficient lag time between the elimination phase of the first peak and the start of absorption of the second peak, two distinct peaks are observed, while in certain other aspects, when the absorption phase of the second peak starts earlier than the completion of the elimination of the first peak, then there may not be two distinct peaks and the peaks may be merged into one peak. In cases when two or more distinct peaks are observed, T₁ would indicate the time to achieve maximum concentration of first peak, T₂ would indicate the time to achieve maximum concentration of second peak and so on. In certain aspects the T₁ ranges from about 0.1 to 5 hours, preferably from about 0.5 to 3.5 hours and T₂ ranges from about 3 to 10 hours, preferably from about 4 to 8 hours, after oral administration. Thus the modified release dosage form of tacrolimus of the present invention comprising at least two dosage units is such that in certain aspects the Tₘₐₓ of first dosage unit is about 0.1 to 5 hours, preferably from about 0.5 to 3.5 hours and Tₘₐₓ of second dosage unit is about 3 to 10 hours, preferably from about 4 to 8 hours. In case of subsequent dosage units, the Tₘₐₓ of subsequent dosage units ranges from about 5 to 16 hours of time of administration.

### DOSAGE FORM OF THE INVENTION

The pulsatile release profiles described in the above paragraphs is provided by the modified release dosage form of tacrolimus described herewith.

The present invention provides a modified release dosage form of tacrolimus comprising at least two dosage units wherein at least one dosage unit is an immediate release dosage unit and the at least second dosage unit is a delayed release dosage unit wherein the immediate release dosage unit comprises tacrolimus and optionally one or more excipients and the at least one delayed release dosage unit comprises tacrolimus and a delayed release material and optionally one or more excipients.

In one aspect of the invention, the modified release dosage form of tacrolimus is a capsule comprising at least two drug-containing "dosage units" wherein each dosage unit within the capsule provides a different drug release profile i.e. immediate release and delayed release. Control of the delayed release dosage unit(s) is accomplished by a polymer coating on the dosage unit(s). Each dosage unit may comprise of plurality of drug-containing beads, pellets, granules, particles or minitablets filled into capsules. Beads, beadlets and pellets can be used interchangeably and has a diameter of about 0.1 to 2 mm or even from 0.3 to 1.5 mm; particles or granules can be of any suitable size and shape, ranging in diameter of about 1 micron to about 0.3 mm; and minitablets has a diameter of 1.5 to 4 mm or even 2 to 4 mm. Drug-containing beads can be produced by applying drug to an inert material/support, e.g., inert sugar beads coated with drug or by creating a "core" comprising both drug and one or more excipients or polymer as known in the art. Drug-containing "granules" and "particles" comprise drug particles that may or may not include one or more additional excipients or polymers. In contrast to drug-containing beads, the drug-containing granules and the drug particles do not contain an inert support. Granules generally comprise drug particles and require further processing and particles which are smaller than granule are generally not further processed. Although beads, granules and particles may be formulated to provide immediate release, beads and granules are generally employed to provide delayed release.

In another aspect of a capsule dosage form, each dosage unit may comprise of compressed or molded tablets, wherein each tablet within the capsule may provide a different drug release profile.

In another aspect, the dosage form is a tablet comprising of "dosage units", each providing a different drug release, i.e immediate release and delayed release, wherein the dosage units are either compacted into a single tablet or they represent integral but discrete segments thereof e.g. a multi-layered tablet. For example, drug-containing particles, granules or beads can be compressed together into a single tablet or multi-layered tablet using conventional tabletting means known in the art. In a further aspect, the dosage form is a coated tablet or an inlay tablet that comprises an inner drug-containing core and at least one drug-containing layer surrounding the inner core, wherein the outer drug-containing layer contains the immediate release amount and the inner drug-containing core comprising of matrix polymer or a polymeric-coating containing the delayed release amounts, for two or more delayed release amounts, layers interposed between the inner core and outer layer contains the various delayed release amounts released after a predetermined time interval of substantially no drug release. In still further aspects, the dosage form is a single or multiple layered osmotic dosage form, the description of which is well known to a person skilled in the art.

In an another aspect, the dosage form of the invention may be in the form of a liquid composition, wherein the first amount of tacrolimus is dissolved and/or dispersed, either alone or in combination with excipient, in a pharmaceutically acceptable vehicle, to form the immediate release pulse; and the second amount of tacrolimus or its pharmaceutically acceptable salt, is also dispersed in the vehicle in the form of granules, particles, beads or pellets, to form the delayed release pulse.

As will be appreciated by those skilled in the art and as described in the pertinent texts and literature, a number of methods are available for preparing drug-containing tablets, beads, granules or particles that provide a variety of drug release profiles. Such methods include, but are not limited to the following: placing the drug within a suitable matrix; mixing the drug with suitable polymers; excipients and solvents and evaporating the solvent to form precipitates or dispersions, coating a drug or drug-containing composition with an appropriate coating material, typically although not necessarily incorporating a polymeric material; mixing or coating a drug or drug-containing composition with an appropriate hydrophilic and/or lipophilic surfactant or additives; granulating the drug with pharmaceutically acceptable carrier or polymer followed by extrusion and spheronization, and forming complexes of the drug with a suitable complexing agent.
(a) Immediate Release Dosage Unit
   The immediate release dosage unit of the dosage form comprises the tacrolimus along with conventional pharmaceutical excipients in the form of beads (beads here refer to non-pareil seeds, eg. sugar, starch, lactose or such other beads with drug as matrix or drug coated on the beads). A preferred method for forming the immediate release drug-containing beads comprises the steps of (a) dissolving tacrolimus along with hydrophilic and/or lipophilic surfactants or other additives including lipophilic additives in a suitable solvent to obtain a clear solution/suspension;
(b) coating the non-pareil seeds with the solution/suspension obtained in step (a); and (c) optionally coating the beads with film forming agents.

The surfactant includes at least one hydrophilic surfactant or lipophilic surfactant or combination thereof. Surfactant with lower HLB value is lipophlilic in nature and surfactant which has higher HLB values is considered to be hydrophilic in nature. The hydrophilic surfactant can be selected from group of anionic, cationic, zwetterionic or and non-ionic surfactant for which HLB scale is not generally applicable. In general, lipophlilic surfactant will have HLB value less than about 10. Mixtures of hydrophilic surfactants or lipophilic surfactants are also within the scope of invention. The amount and choice of specific hydrophilic or lipophlilic surfactant or combination thereof will be appreciated by a person skilled in the art to enable the dosage form of the present invention.

The suitable surfactant includes but is not limited to polyethoxylated fatty acids, polyethylene glycol fatty acid diesters, polyethylene glycol fatty acid Mono- and Di-ester mixtures, polyethylene glycol glycerol fatty acid esters, alcohol-oil transesterification products, polyglycerized fatty acids, propylene glycol fatty acid esters, mixture of propylene glycol esters-glycerol esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-polyoxypropylene block copolymers, sorbitan fatty acid esters, lower alcohol fatty acid esters and ionic surfactants. The ionic surfactants include fatty acid salts (sodium caprylate, sodium dioctyl sulfosuccinate, sodium lauryl sulfate sodium laurate, sodium palmitate etc.), bile salts (sodium cholate, sodium glycocholate, etc.), phospholipids (egg/soy lecithin, lysophosphatidylcholine, phosphatidyl glycerol), phosphoric acid esters (esterification productof fatty alcohol or fatty alcohol ethoxylates with phospphoic acid or anhydrides), carboxylates (sodium strearyl fumarate, citric acid esters of mono-,diglycerids), sulfates and sulfonates (acyl taurates, alkyl glyceryl ether sulfonates, octyl sulfosuccinate disodium). The cationic surfactants include but not limited to hexadecyl triammonium bromide, decyl trimethyl ammonium bromide, alkylpyridinium salts, betaines, lauryl beanie polyoxyethylene-15 coconut amine.

Preferably, the surfactant is selected from group of PEG4-100 monolaurate, PEG4-100 monooleate, PEG4-100 monosterate, PEG4- dioleate, PEG8 dilaurate, PEG12 distearate, PEG4-150 mono,dilaurate, PEG20 glyceryl state, PEG35 castor oil, hydrogenated castor oil e.g. PEG40 hydrogenated castor oil, PEG5 hydrogenated castor oil, PEG9 hydrogenated castor oil, PEG6 peanut oil, PEG 20 corn glycerides, polyglyceryl-2-stearate, polyglyceryl-2- oleate, polyglyceryl-10 laurate, polyglyceryl-2- dioleate, propylene glycol monocaprylate propylene glycol dilaurate, propylene glycol monolaurate, glycerol monooleate, glycerol monolinolate, glyceryl laurate, distearin, Peg24 cholesterol ether solulan, PEG-20 sorbitan monooleate, PEG-20 sorbitan monolaurate, PE-2 oleyl ether, PEG-9 lauryl ether, sucrose monostearate, sucrose monolaurate, poloxamer, sobitan monopalmitate, sorbitan monooleate, sodium dioctyl sulfosuccinate and sodium lauryl sulfate.

Lipophilic additives used in the present invention include lipophlilic surfactant or triglycerides. Triglycerides are those which solidify at ambient room temperature, with or without addition of appropriate additives, or those which in combination with particular surfactants and/or active ingredients solidify at room temperature. Fractionated triglycerides, modified triglycerides, synthetic triglycerides, and mixtures of triglycerides are also within the scope of the invention.

Examples of triglycerides that can be used in the present invention include almond oil, caster oil, cocoa butter, coconut oil, corn oil, grape seed oil, groundnut oil, linseed oil, olive oil, palm oil, peanut oil, poppy seed oil, rapeseed oil, sal fat, sesame oil, shark liver oil, soybean oil, sunflower oil, tobacco seed oil, wheat germ oil, hydrogenated caster oil, hydrogenated coconut oil, hydrogenated vegetable oil, glyceryl tricaprate, glyceryl tripalmitate, glyceryl tristearate, glyceryl trioleate, glyceryl 1,2-caprylate-3-linolate, glyceryl 1,2-caprate-3-stearate, glyceryl 1,3-palmitate-2-butyrate. Preferred triglycerides include vegetable oils, fish oils, animal fats, hydrogenated vegetable oils, partially hydrogenated vegetable oils, medium and long-chain triglycerides, and structured triglycerides.

Several commercial surfactants which contain small to moderate amounts of triglycerides, typically as a result of incomplete reaction of a triglyceride starting material in, for example, a trans-esterification reaction, are also within the scope of the invention. Such commercial surfactants may be suitable to provide all or part of the triglyceride component for the compositions of the present invention. Examples of such surfactants containing triglycerides include some members of the surfactant families Gelucires (Gattefosse), Maisines (Gattefosse), and lmwitors (Hüls). Specific examples of these compositions are: Gelucire 44/14 (saturated polyglycolized glycerides); Gelucire 50/13 (saturated polyglycolized glycerides); Gelucire 53/10 (saturated polyglycolized glycerides); Gelucire 33/01 (semi-synthetic triglycerides of C8-C18 saturated fatty acids); Gelucire 39/01 (semi-synthetic glycerides); other Gelucires, such as 37/06, 43/01, 35/10, 37/02, 46/07, 48/09, 50/02, 62/05, etc.; Maisine 35-I (linoleic glycerides); and Imwitor 742 (caprylic/capric glycerides). Still other commercial surfactants having significant triglyceride content are known to those skilled in the art.

Preferred hydrophilic surfactant is Vitamin E TPGS and lipophilic surfactants are glycerol monooleate or propylene glycol monolaurate. Vitamin E TPGS is a water-soluble form of natural source vitamin E. It is prepared by esterifying the acid group of crystalline d-alpha-tocopheryl acid succinate with polyethylene glycol 1000. Vitamin E TPGS is very stable and does not hydrolyze under normal conditions. Vitamin E TPGS is used for enhancing the bioavailability of fat soluble drug that are difficult to absorb. Glyceryl monooleate (sold as Peceol) is a mixture of the monoglycerides, mainly glyceryl monooleate together with variable quantities of diglycerides and triglycerides. It is obtained by partial glycerolysis of vegetable oil that consists mainly of triglycerides of oleic acid, or by esterification of glycerol with oleic acid of vegetable or animal origin. It has a HLB value of 3 to 4. Propylene glycol monolaurate (sold as Lauroglyco190^{®} and Lauroglycol FCC^{®}) is a mixture of the propylene glycol mono and di-esters of lauric acid, both having low HLB values between 4 to 5 and are used in formulations to enhance the bioavailability of poorly soluble drugs. Hydrophilic and lipophilic surfactants or lipophilic additives are preferably used in range of about 0.5 % to about 40% by weight and more preferably in the range of about 1% to about 20% by weight of the total weight of the dosage form.

In another preferred aspect, immediate release dosage unit is prepared by mixing tacrolimus and a suitable polymer or a water-soluble carrier in a suitable solvent optionally along with other excipients and either (i) coating or adsorbing this solution on granules, powder, beads or pellets, which may be optionally coated with film forming materials or (ii) evaporating the solvent to obtain a dried mixture which may be further subjected to size reduction to prepare solid dispersions. Solid dispersions or fine particles of tacrolimus thus obtained may also be prepared by any other techniques well known in the art. The beads may be then filled in capsules or sachets to be included in the dosage form.

In another aspect the immediate release dosage unit is in the form of tablets (e.g minitablets filled into capsule) and is prepared by compressing a drug-containing blend, e.g., blend of granules, prepared using a direct blend, wet-granulation or dry-granulation process as known in the art in a tablet compression machine.

Thus the immediate release dosage unit is a solid dispersion of tacrolimus in a water-soluble carrier coated or adsorbed on an inert material selected from beads, non-pareil seeds, granules, pellets, particles and core tablets.

Useful water-soluble carrier or polymer includes but are not limited to polyethylene glycols, polyoxyethylene oxides, polaxomers, polyoxyethylene stearates, poly-epsilon caprolactone, polyglycolized glycerides, polyvinyl pyrrolidones, polyvinyl-polyvinyl acetate copolymers (PVP-PVA), polyvinyl alcohol (PVA), polymethacrylic polymers, cellulose derivatives including hydroxypropyl methylcellulose (HPMC), hydroxypropyl cellulose (HPC), methyl cellulose, sodium carboxymethyl cellulose, hydroxyethyl cellulose, pectins, cyclodextrins, galactomannans, alginates, carragenates, xanthan gums and mixtures thereof, preferably the water-soluble carrier is HPMC. HPMC or Hypromellose (methocel; methocel E6 Premium LV) is propylene glycol ether of methylcellulose. Typical viscosity value for 2% aqueous solution of Methocel E6 Premium LV is 6 mPas at 20ºC. When used in the dosage form, the weight ratio of tacrolimus to the water-soluble carrier is in the range of from about 2 : 1 to about 1 : 10; preferably from about 1 : 1 to about 1 : 4.

Solvents that can be used to dissolve the drug, carrier and excipients include acetone, ethanol, isopropanol, ethyl acetate, and mixtures of two or more.

### (b) Delayed Release Dosage Unit

The delayed release dosage units of the present invention can be prepared by further coating a drug or drug-containing beads, granules, pellets, particles or tablet prepared as above with a delayed release material. The delayed release material is selected from group of bioerodible polymers, water soluble or water-insoluble polymers, enzymatic degradable and/or pH dependant polymers, including the enteric materials. The coating weight, type or relative amount of coating material may be readily determined by those skilled in the art by evaluating individual release profiles for tablets, beads and granules prepared with different quantities of various coating materials.

Thus the delayed release dosage unit is a solid dispersion of tacrolimus in a water-soluble carrier coated or adsorbed on an inert material selected from beads, non-pareil seeds, granules, pellets, particles and core tablets which is further coated with an enteric coating material.

Thus a modified release dosage form according to the invention releases tacrolimus in a pulsatile manner and provides an extended therapeutic action of tacrolimus. In one aspect the modified release dosage form of tacrolimus comprises at least two dosage units wherein at least one dosage unit is an immediate release dosage unit and the at least second dosage unit is a delayed release dosage unit wherein the immediate release dosage unit comprises tacrolimus or an analogue or salt thereof and optionally one or more excipients and the at least one delayed release dosage unit comprises tacrolimus or analogue or salt thereof and a coating and optionally one or more excipients wherein the coating is an enteric coating or pH dependent coating.

Suitable enteric coating material is selected from the group consisting of cellulosic polymers, acrylic acid polymers and copolymers, vinyl polymers and copolymers and enzymatically degradable polymers or a mixture thereof, maleic acid-based polymers and copolymers, polyvinyl derivatives, zein, shellac, cellulose esters, cellulose acetate phthalates and ethyl cellulose.

More examples of the enteric coating material that can be used in the present invention includes polyacrylamides, phthalate derivatives such as acid phthalate of carbohydrates including amylase acetate phthalate, cellulose acetate phthalate, cellulose acetate terephthalate, cellulose acetate isophthalate, other cellulose ester phthalates, cellulose ether phthalates, hydroxypropyl cellulose acetate phthalate, hydroxypropyl ethyl cellulose phthalate, hydroxypropyl methylcellulose phthalate (HPMCP), methyl cellulose phthalate, methyl cellulose acetate phthalate, polyvinyl acetate phthalate, polyvinyl acetate hydrogen phthalate, sodium cellulose acetate phthalate, starch acid phthalate; phthalate of other compounds including polyvinyl acetate phthalate (PVAP); other cellulose derivatives including hydroxypropyl methylcellulose acetate succinate (HPMCAS), carboxymethyl cellulose, cellulose acetate trimelliate, alginates; carbomers; polyacrylic acid derivatives such as acrylic acid and acrylic ester copolymers, polymethacrylic acid and esters thereof, polyacrylic methacrylic acid copolymers, methacrylic acid copolymers (for example Eudragit L and Eudragit S); styrene-maleic acid dibutyl phthalate copolymer, styene and maleic acid copolymers; shellac, starch glycolate; polacrylin; vinyl acetate and crotonic acid copolymers and mixtures thereof; in particular poly (methyl acrylate, methyl methacrylate, methacrylic acid).

In preferred aspect, the coating material is poly (methyl acrylate, methyl methacrylate, methacrylic acid) 7:3:1, known as Eudragit FS 30D.
The coating can also be pH independent coating, water soluble or water-miscible coating and water-insoluble or hydrophobic coating; suitable material is selected from a group consisting of ethyl cellulose, cellulose acetate, cellulose nitrate, cellulose derivatives selected from the group of hydroxypropyl methylcellulose, hydroxypropyl cellulose, methyl cellulose, sodium carboxymethyl cellulose, hydroxyethyl cellulose, poloxamers, polyethylene stearates, poly-s-caprolactone, polyvinyl pyrrolidone, polyvinylpyrrolidone-polyvinylacetate copolymer, polymethacrylic polymers and polyvinyl alcohol, polyethylene oxide and mixtures thereof.

The delayed release dosage unit is coated with the coating material which is the delayed release material from up to at the most 40%, up to at the most 20%, up to at the most 10% of weight gain from the weight of the dosage unit before coating.

In another aspect the delayed release dosage unit may be formulated by mixing or dispersing the tacrolimus with a suitable delayed release material in the form of matrix, the delayed release material is in the form of a matrix is selected from the group consisting of water-miscible polymers, water-insoluble polymers, acrylic and methacrylic acid based polymers and copolymers, hydroxypropyl methyl cellulose, polyvinyl pyrrolidone, and ethyl cellulose, oils and oily materials, gums, substituted or unsubstituted hydrocarbons, fatty acids, fatty alcohols, glyceryl esters of fatty acids, minerals, vegetable oils and waxes.
Suitable delayed release material being selected from a group of polymers or copolymers of cellulose, cellulose ester, acrylic acid, methacrylic acid, methyl acrylate, ethyl acrylate, and vinyl or enzymatically degradable polymers or copolymers as described herein. These materials are particularly useful for providing a delayed release matrix. Fatty compounds for use as a matrix material include, but are not limited to, lipid, waxes (e.g. carnauba wax) and glycerol tristearate. After mixing the active ingredient with the matrix material, the mixture can be formulated as granules, pellets, beads, particles or compressed into tablets.

Preferable suitable coating materials or matrix materials for effecting delayed release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename Eudragit® (Rohm Pharma; Westerstadt, Germany), including Eudragit® L30D-55 and L100-55 (soluble at pH 5.5 and above), Eudragit® L-100 (soluble at pH 6.0 and above), Eudragit® S (soluble at pH 7.0 and above, as a result of a higher degree of esterification), and Eudragits® NE, RL, FS 30D and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, arabinogalactose, amylase, chondroitin sulfate, dextran, galactomannan, xylan and guar gum, locust-bean gum, gum tragacanth and Karaya gum; and shellac. Combinations of different coating materials may also be used. Multi-layer coatings using different polymers may also be applied. The amount and type of delayed release material to prevent the release of therapeutic agent in gastric pH can be determined by a person skilled in the art. In the present invention delayed release material can be in range of from about 1% w/w to about 50% w/w of the dosage unit.

### Film Forming Polymers

The immediate release and/or the delayed release dosage units is coated with a film-forming material selected from the group consisting of water-soluble or water-insoluble polymer(s), natural, semisynthetic or synthetic polysaccharides. These film forming materials used in this invention include but is not limited to water soluble or water insoluble polymers such as cellulose derivatives, acrylic polymers or copolymers, vinyl polymer and other high molecular polymer derivatives or synthetic polymers such as methylcellulose, hydroxypropylcellulose, hydroxypropylmethylcellulose, ethylcellulose, cellulose acetate, polyvinyl pyrrolidone, polyvidone acetate, polyvinyl acetate, polymethacrylaes and ethylene-vinyl acetate copolymer or a combination thereof, other natural, semisynthetic, or synthetic polysaccharides, such as, alginic acid, alkali metal and ammonium salts thereof, carrageenans, galactomannans, tragacanth, agar-agar, gum arabicum, guar gum, xanthan gum, starches, pectins, such as sodium carboxymethylamylopectin, chitin derivates such as chitosan, polyfructans, inulin; polyacrylic acids and the salts thereof. Preferred film-forming polymers are polyvinyl alcohol, polyvinylpyrrolidone, vinylpyrrolidone-vinyl acetate copolymer and hydroxyalkylcelluloses such as hydroxyethyl cellulose, hydroxypropyl cellulose. The type and amount of film forming material is determined by the person skilled in the art. The film forming material can be coated in range of from about 1% to about 30% w/w of the dosage form.

In certain aspects, both immediate release and delayed release dosage unit are coated with film forming polymer. Delayed release dosage units are further coated with delayed release material for producing the effect of delayed release.

Coating is carried out by various methods such as conventional coating pan, an airless spray technique, fluid bed coating and/or press coating. Coating layer of tacrolimus containing solution, film forming material and delayed release material may optionally comprise appropriate excipients which improve the property of coating layers and such ingredients include but are not limited to fillers, plasticizers, anti-adhesives, pigments, coloring agents, binders, stabilizing agents, solubilizers, pore formers or any other pharmaceutically acceptable excipients known to the art, used either alone or in combination thereof. It will be appreciated that certain excipients used in the present composition can serve more than one purpose.

Examples of typical plasticizers used include, but are not limited to, phthalic acid esters, diethyl phthalate, dibutyl phthalate, citric acid esters, butyl citrate, triethyl acetyl citrate, monoglycerides, triacetin, dibutyl sebacate and polyethylene glycol.

### Other Excipients

Other excipients or additives which may be included in the dosage of the present invention are fillers, diluents, binders, disintegrants, stabilizers, surfactants, wetting agents, buffering agents, preservatives, absorption enhancers, wicking agents, glidants, lubricants etc.

Diluents, also known as fillers, typically function as carriers and increase the bulk of the pharmaceutical composition so that a practical size is provided for manufacturing, such as compression of tablets and formation of beads or granules. Suitable diluents include, for example, lactose, sucrose, mannitol, sorbitol, microcrystalline cellulose, powdered cellulose, dry starch, hydrolysed starches, pregelatinized starch, dicalcium phosphate, calcium sulfate and titanium dioxide.

Binders are used to impart cohesive qualities to a system, to ensure its intactness. Suitable examples include starch, pregelatinized starch, polyvinylpyrrolidone, ethylcellulose, methylcellulose, microcrystalline cellulose, derivatized cellulose, such as carboxymethyl cellulose, sodium carboxymethyl cellulose, hydroxyethyl cellulose, hydroxypropyl methylcellulose, and hydroxypropyl cellulose, polyethylene glycol, and waxes, natural and synthetic gums such as acacia, tragacanth, sodium alginate and veegum.

Disintegrants are used to facilitate disintegration of the system after administration. Suitable examples include starch, sodium starch glycolate, carbopol, various celluloses, sodium carboxymethyl cellulose, clays, gums such as agar, arabic, guar, locust bean and crosslinked polymers such as crosslinked PVP and crosslinked carboxymethyl cellulose.

Lubricants prevent sticking and facilitate smooth manufacturing of a system. Suitable examples include magnesium stearate, stearic acid and its pharmaceutically acceptable alkali metal salts, calcium stearate, sodium stearate, Cab-O-Sil, Syloid, polyethylene glycol, magnesium lauryl sulfate, sodium stearyl fumarate, vegetable oil and talc.

Absorption enhancers assist in increasing the absorption of active agent molecules through the gastrointestinal mucosa and improving their bioavailability. Absorption enhancers which may be used belong to categories such as cell envelope disordering compounds, solvents, steroidal detergents, bile salts, chelators, surfactants, non-surfactants, fatty acids etc. Examples include chelators such as EDTA, citric acid, sodium salicylate; surfactants such as sodium lauryl sulphate, benzalkonium chloride, polyoxyethylene, 23-lauryl ether; bile salts such as sodium deoxycholate, sodium glycocholate, sodium taurocholate; fatty acids such as oleic acid, capric acid, lauric acid; non-surfactants such as cyclic ureas, cyclodextrins; and others such as polysorbates, aprotinin, azone, alkyl glycosides, chitosan, menthol, dextran sulfate etc.

In certain aspects the modified release dosage form of the present invention comprises (a) between about 0.05% w/w and about 50.0% w/w of tacrolimus; (b) between about 1.0% w/w and about 40.0% w/w of hydrophilic surfactant; (c) between about 0.1% w/w and about 30.0% w/w of lipophilic additive; and (d) between about 1.0% w/w and about 30% w/w of delayed release material of the total weight of the dosage form.

In certain other aspects the modified release dosage form of the present invention comprises (a) between about 0.05% w/w and about 50.0% w/w of tacrolimus; (b) between about 1.0% w/w and about 40.0% w/w of hydrophilic surfactant; (c) between about 0.1% w/w and about 30.0% w/w of lipophilic additive; (d) optionally between about 0.01% w/w and about 10.0% w/w of hydrophilic polymer; (e) between about 1.0% w/w and about 30% w/w of delayed release material and (f) between about 1.0% w/w and about 90.0% w/w of other excipients.

In certain aspects the modified release dosage form of the present invention comprises (a) between about 0.05% w/w and about 50.0% w/w of tacrolimus; (b) between about 1.0% w/w and about 40.0% w/w of vitamin E TPGS; (c) between about 0.1% w/w and about 30.0% w/w of lipophilic additive selected from glycerol monooleate or propylene glycol monolaurate; (d) optionally between about 0.01% w/w and about 10.0% w/w of hydroxylpropyl methyl cellulose; (e) between about 1.0% w/w and about 30% w/w of delayed release material selected from methacrylic acid co-polymer or acrylate polymer and (f) between about 1.0% w/w and about 90.0% w/w of other excipients.

In certain aspects the modified release dosage form of the present invention comprises (a) between about 0.05% w/w and about 50.0% w/w of tacrolimus; (b) between about 0.01% w/w and about 50.0% w/w of water-soluble carrier; and (c) between about 1.0% w/w and about 30% w/w of delayed release material of the total weight of the dosage form.

In certain other aspects the modified release dosage form of the present invention comprises (a) between about 0.05% w/w and about 50.0% w/w of tacrolimus; (b) between about 0.01% w/w and about 50.0% w/w of water-soluble carrier; (c) between about 0.01% w/w and about 20.0 % w/w of surfactant; (d) between about 1.0% w/w and about 30% w/w of delayed release material and (e) between about 1.0% w/w and about 30.0% w/w of other excipients of the total weight of the dosage form.

In certain aspects the modified release dosage form of the present invention comprises (a) between about 0.05% w/w and about 50.0% w/w of tacrolimus; (b) between about 0.01% w/w and about 50.0% w/w of hydroxypropyl methyl cellulose; (c) between about 0.01% w/w and about 20.0 % w/w of surfactant selected from sodium lauryl sulfate or dioctyl sodium sulfosuccinate; (e) between about 1.0% w/w and about 30% w/w of delayed release material selected from methacrylic acid co-polymer or acrylate polymer and (f) between about 1.0% w/w and about 30.0% w/w.of other excipients of the total weight of the dosage form.

A method for preparation of the modified release dosage form of tacrolimus comprises the steps of: a) formulating a first amount of tacrolimus optionally with one or more excipients to form an immediate release dosage unit; b) formulating a second amount of tacrolimus with a delayed release material to form a delayed release dosage unit; c) optionally formulating a third amount of tacrolimus with a delayed release material to form a second delayed release dosage unit; and d) incorporating all the dosage units together to form the dosage form of the present invention.

**EXAMPLES:** The following non-limiting examples illustrate an aspect of the invention and should not be construed to limit the scope of the invention. Any modifications of the method of invention are within the spirit or scope of the invention.

### Example 1

Dosage forms were prepared containing immediate release component and modified release component as per the invention. The steps involved dissolving hydrophilic surfactant (vitamin E TPGS), tacrolimus and lipophilic surfactant (glycerol monooleate) in a suitable solvent (isopropyl alcohol) and coating it on non-pareil seeds. These obtained pellets were divided into two parts, one part, which formed the immediate release dosage unit, was further coated with a film forming polymer solution and the second part, which formed the delayed release dosage unit, were coated with an enteric material using the composition as given in Table 1, using a suitable coating equipment. Pellets of immediate release and delayed release components corresponding to their desired amount were then filled into capsules. In some formulation, second part of the composition which formed delayed release dosage unit may be coated with film coating composition prior to enteric coating.

**Table 1: Compositions of tacrolimus based on mixture of hydrophilic surfactant and lipophilic surfactant**

| Ingredients | Composition/ (Quantity in mg) | | | | | | |
|---|---|---|---|---|---|---|---|
| | A | B | C | D | E | F | G |
| **Immediate release dosage unit** | | | | | | | |
| Tacrolimus | 1.60 | 1.60 | 2.50 | 2.50 | 2.50 | 2.50 | 2.50 |
| Isopropyl alcohol* | 26.14 | 96.00 | 42.5 0 | 28.69 | 42.59 | 40.98 | 40.98 |
| Vitamin E TPGS | 24.13 | 128.00 | 39.41 | 26.52 | 39.41 | 37.88 | 37.88 |
| Glycerol monooleate | 9.05 | - | - | 10.04 | - | 14.33 | 14.33 |
| Propylene glycol monolaurate | - | - | 14.78 | - | 14.77 | - | - |
| Propylene Glycol | - | 160.00 | - | - | - | - | - |
| Purified Water* | - | - | - | - | - | - | - |
| Colloidal Silicondioxide | - | - | 23.14 | 10.34 | 23.148 | 14.76 | 14.76 |
| Non-Pareil Seeds | q.s | q.s | 250.00 | 196.36 | 250.00 | 280.51 | 277.77 |
| **Film coating:** | | | | | | | |
| Hydroxypropyl methyl-cellulose | - | - | - | 5.00 | 6.59 | 7.81 | 6.94 |
| Purified water* | - | - | - | q.s. | q.s. | q.s. | q.s. |
| **Delayed release dosage unit:** | | | | | | | |
| Tacrolimus | 3.40 | 3.40 | 2.50 | 2.50 | 2.50 | 2.50 | 2.50 |
| Isopropyl alcohol* | 5.56 | 204.00 | 42.5 0 | 28.69 | 42.5 9 | 40.98 | 40.98 |
| Vitamin E TPGS | 51.27 | 272.00 | 39.41 | 26.52 | 39.41 | 37.88 | 37.88 |
| Glycerol monooleate | 19.24 | - | - | 10.03 | - | 14.33 | 14.33 |
| Propylene glycol monolaurate | - | - | 14.7 8 | - | 14.77 | - | - |
| Propylene glycol | - | 340.00 | - | - | - | - | - |
| Purified water* | - | - | - | - | - | - | - |
| Colloidal silicon dioxide | - | - | 23.14 | 10.3347 | 23.148 | 14.76 | 14.76 |
| Non-Pareil Seeds | q.s | q.s | 250. 00 | 196.36 | 250. 00 | 280.5 1 | 277.7 7 |
| Hydroxypropyl methylcellulose | - | - | - | - | - | 7.81 | - |
| Purified water* | - | - | - | - | - | 64.28 | - |
| **Enteric coating:** | | | | | | | |
| Methacrylic acid copolymer | 11.69 | 58.42 | - | 60.92 | - | - | 52.33 |
| Acrylate polymer (Dry powder wt) | | | 98.7 | | 105.54 | 76.41 | |
| Triethyl citrate | 2.98 | 14.88 | - | 15.50 | - | 3.34 | 13.31 |
| Acetone* | 60.78 | 303.88 | - | 316.81 | - | - | 272.18 |
| Isopropyl alcohol* | 52.06 | 261.32 | - | 625.88 | - | - | 537.71 |
| Purified water* | 10.63 | 53.13 | - | 55.38 | - | 151.7 | 47.57 |
| Talc | 6.44 | 32.22 | - | 33.56 | - | | 28.83 |

| | | | | | | | |
|---|---|---|---|---|---|---|---|
| *Not present in final composition | | | | | | | |

The dosage forms were tested for *in-vitro* dissolution properties using USP dissolution apparatus II by pH changed method and the samples were analyzed by HPLC.

**Table 2: In-vitro dissolution profile**

| **Medium** | **Time (Hrs )** | **Composition / (% Drug Release)** | | | |
|---|---|---|---|---|---|
| | | D | E | F | G |
| pH 1.2 | 0 | 0 | 0 | 0 | 0 |
| pH 1.2 | 2 | 32.6 | 30.9 | 32.0 | 37.9 |
| pH 4.5 | 3 | 32.8 | 38.4 | 35.0 | 46.4 |
| pH 6.8 | 4 | 51.1 | - | 53.0 | - |
| pH 7.4 | 5 | 102.5 | - | 93.6 | 46.9 |
| pH 7.4 | 6 | - | 35.3 (at pH 6.8) | 96.2 | 74.0 |
| pH 7.4 | 7 | - | 95.3 | 99.8 | 87.4 |
| pH 7.4 | 8 | - | 94.5 | 101.4 | 89.1 |
| pH 7.4 | 9 | - | - | - | 90.2 |
| pH 7.4 | 10 | - | - | - | 91.0 |
| pH 7.4 | 11 | - | - | - | 91.1 |
| pH 7.4 | 12 | - | - | - | 91.2 |

### Example 2

Dosage forms were prepared containing immediate release component and modified release component as per the invention. The steps involved dissolving hydrophilic surfactant (sodium lauryl sulfate, dioctyl sodium sulfosuccinate), tacrolimus, water-soluble carrier and other excipients in a suitable solvent (ethanol, dichloromethane or mixture thereof) to obtain clear solution. The above obtained solution is coated over non-pareil seeds. These pellets were divided into two parts, one part, which formed the immediate release dosage unit, was further coated with a film forming polymer solution and the second part, which formed the delayed release dosage unit, were coated with an enteric material using the composition as given in Table 3, using a suitable coating equipment. Pellets of immediate release and delayed release components corresponding to their desired amount were then filled into capsules. In some formulation, second part of the dosage form which formed the delayed release dosage unit may be coated with film coating prior to enteric coating.

**Table 3: Solid Dispersion Compositions of Tacrolimus**

| **Ingredients** | **Composition/ (Quantity in mg)** | | | | | | |
|---|---|---|---|---|---|---|---|
| | H | I | J | K | L | M | N |
| **Immediate release dosage unit:** | | | | | | | |
| Tacrolimus | 2.50 | 2.50 | 2.50 | 2.00 | 2.00 | 0.40 | 0.20 |
| Hydroxypropyl methylcellulose | 2.50 | 2.50 | 2.50 | 2.00 | 2.00 | 0.40 | 0.20 |
| Sodium Lauryl Sulfate | - | 0.03 | - | - | - | - | - |
| Dioctyl sodium sulfosuccinate | - | - | 0.25 | - | - | - | - |
| Ethanol* | 76.65 | 76.65 | 75.00 | 120.00 | 120.00 | 24.00 | 12.00 |
| Dichloromethane* | 38.33 | 38.33 | 37.50 | - | - | - | - |
| Lactose Monohydrate | - | - | - | - | 1.85 | 0.37 | 0.19 |
| Non-Pareil Seeds | 250.00 | 250.00 | 250.00 | 200.00 | 200.00 | 40.00 | 20.00 |
| **Delayed release dosage unit:** | | | | | | | |
| Tacrolimus | 2.50 | 2.50 | 2.50 | 3.00 | 3.00 | 0.60 | 0.30 |
| Hydroxypropyl methylcellulose | 2.50 | 2.50 | 2.50 | 3.00 | 2.00 | 0.60 | 0.30 |
| Sodium Lauryl Sulfate | - | 0.03 | - | - | - | - | - |
| Dioctyl sodium sulfosuccinate | - | - | 0.25 | - | - | - | - |
| Ethanol* | 76.65 | 76.65 | 75.00 | 180.00 | 120.00 | 36.00 | 18.00 |
| Dichloromethane* | 38.3 3 | 38.3 3 | 37.50 | - | - | - | - |
| Lactose Monohydrate | - | - | - | - | 1.85 | 0.56 | 0.28 |
| Non-Pareil Seeds | 250.00 | 250.00 | 250.00 | 300.00 | 200.00 | 60.00 | 30.00 |
| **Enteric Coating:** | | | | | | | |
| Methacrylic acid copolymer | 75.00 | 75.00 | 76.57 | 91.00 | 61.75 | 12.35 | 6.18 |

| (Dry powder wt) | | | | | | | |
|---|---|---|---|---|---|---|---|
| Talc | - | - | - | q.s. | q.s. | q.s. | q.s. |

| | | | | | | | |
|---|---|---|---|---|---|---|---|
| *Not present in final composition | | | | | | | |

The dosage forms were tested for in-vitro dissolution prop erties using USP dissolution apparatus II in pH changed method and the samples were analyzed by HPLC

**Table 4: In-vitro dissolution profile**

| **Medium** | **Time (Hrs)** | **Formulation (% Drug Release)** | | | | |
|---|---|---|---|---|---|---|
| | | H | I | J | K | L |
| pH 1.2 | 0 | 0 | 0 | 0 | 0 | 0 |
| pH 1.2 | 2 | 41.13 | 47.4 | 37.90 | 19.62 | 30.25 |
| pH 4.5 | 3 | 43.15 | 59.02 | 43.20 | 22.32 | 30.53 |
| pH 6.8 | 6 | 45.3 | 59.48 | 43.30 | 20.90 | 32.28 |
| pH 7.4 | 7 | 92.3 | 99.13 | 83.40 | 106.54 | 102.73 |
| pH 7.4 | 8 | - | - | 90.60 | - | - |
| pH 7.4 | 9 | - | - | - | - | - |
| pH 7.4 | 12 | - | - | - | - | - |

### Example 3: Pharmacokinetic studies:

(a) A study was designed in a small group of healthy human volunteers to evaluate the pharmacokinetic profiles of a single dose oral administration of tacrolimus compositions E and J of the present invention (5mg dosed once-a-day) ; Test Products T₁ and T₂ respectively and compare it with the pharmacokinetics of a commercially available conventional immediate release product, Prograf®, (2.5 mg dosed twice-a-day); Reference product (R).
Study design: An open-label, randomized, fasted, single dose pharmacokinetic study. Healthy human volunteers were subjected to overnight fasting prior to the dosing. Formulations were given to individual volunteer with 250 ml water. Blood samples were collected pre-dose and after pre-determined time intervals (0.5, 1.0, 1.5, 2.0, 4.0, 8.0, 9.0, 10.0, 11.0, 12.0, 12.5, 13.0, 13.5, 14.0, 16.0, 20.0, 22.0, 24.0, 36.0 hours after dosing). Standard diet was given to the volunteers during the study. Plasma analysis was done using validated analytical method to determine pharmacokinetic parameters viz Cmax, Tmax, C₄, C₂₄, AUC₀₋ₜ and AUC_{0-inf} and the data is presented in Table 5.

**Table 5: Comparative Pharmacokinetic Data of Single Dose Administration of Tacrolimus Compositions**

| **Sample** | **C₄** | **C₂₄** | **AUC₀₋ₜ (Ln)** | **AUC_{0- inf} (Ln)** | **Cₘₐₓ (Ln)** | **T₁ (hr)** | **T₂ (hr)** |
|---|---|---|---|---|---|---|---|
| Reference (R) | 7.14 | 3.17 | 141.78 | 177.22 | 14.14 | 1.5 | 14.0 |
| Composition E* (T₁) | 13.81 | 4.04 | 200.37 | 275.48 | 18.75 | 1.0 | 4.0 |
| Composition J* (T₂) | 10.31 | 3.44 | 198.12 | 271.51 | 17.85 | 1.0 | 8.0 |

| | | | | | | | |
|---|---|---|---|---|---|---|---|
| * Note compositions E & J are the modified release once-a-day dosage form of tacrolimus of the present invention described in examples 1 and 2 respectively. | | | | | | | |

As seen the AUC_{0-inf} T₁/R value is at 1.55 and T₂/R value is at 1.53, indicating a high bioavailability for test compositions. The C₂₄ values were comparable for test and reference products.
(b) In a separate human cross-over study, 30 fasted healthy volunteers were dosed once with 5 mg tacrolimus composition K; Test product, (T) of the present invention and the pharmacokinetic profiles were compared with (i) a commercially available immediate release product dosed 2.5 mg b.i.d; Prograf®, Reference (R₁) and (ii) a commercially available prolonged release product dosed 5 mg once-a-day; Advagraf®, Reference (R₂). Pharmacokinetic assessments were done similar to that described in (a) above and the data is presented in Table 6 below.

**Table 6: Comparative Pharmacokinetic Data of Single Dose Administration of Tacrolimus Compositions**

| **Sample** | **C₄** | **C₂₄** | **AUC₀₋ₜ (Ln)** | **AUC₀₋ inf (Ln)** | **Cₘₐₓ (Ln)** | **Tₘₐₓ (hr)** |
|---|---|---|---|---|---|---|
| Composition K (T) | 6.6 | 4.63 | 172.16 | 228.94 | 8.25 | 8.0 |
| Reference (R₁) | 8.93 | 4.31 | 172.58 | 222.36 | 15.57 | 2.0 |
| Reference (R₂) | 10.62 | 3.07 | 149.41 | 207.46 | 11.03 | 2.0 |

As seen from the data above, the modified release composition of the present invention administered once-a-day is bioequivalent in terms of AUC₀₋ₜ and AUC_{0-inf} with a T/R₁ value of about 1.0 to the immediate release commercially available product administered two times a day and is better than the commercially available prolonged release product also administered once-a-day by having a relative bioavailability based on AUC₀₋ₜ (T/R₂) 115%. The C₂₄ value of the test composition was comparable to that of the commercially available immediate release product and better than the commercially available prolonged release product by a factor of 1.5. The estimated steady-state fluctuation or % Flux values for R₁ was 33.42 ± 9.21, that for R₂ was 26.47 ± 7.32, and that for the test composition T was 13.75 ± 5.43.

The examples provided above are not meant to be exclusive. Many other variation of the present invention would be obvious to those skilled in the art, and are contemplated to be within the scope of the appended claims.

## Claims

1. A modified release dosage form of tacrolimus that releases two or more amount of tacrolimus upon oral administration wherein the first amount of tacrolimus is released substantially immediately followed by a time interval during which substantially no amount of tacrolimus is released from the dosage form, after which the second amount of tacrolimus is released and further wherein the dosage form optionally comprises an additional amount of tacrolimus.

2. A modified release dosage form of tacrolimus according to claim 1 wherein the first amount of tacrolimus is present in immediate release dosage unit and the second amount of tacrolimus is present in delayed release dosage unit.

3. A modified release dosage form of tacrolimus according to claim 2 wherein the first amount of tacrolimus is present in an amount of about 10% w/w to about 70% w/w of the total amount of tacrolimus and the second amount of tacrolimus is present in an amount of about 30 % w/w to about 90% w/w of the total amount of tacrolimus.

4. A modified release dosage form of tacrolimus according to claim 1 wherein the first amount of tacrolimus is released substantially immediately within 0-2 hours followed by a time interval ranging from about 1-10 hours during which substantially no amount of tacrolimus is released from the dosage form, after which a second amount of tacrolimus is released wherein said second amount is released either immediately within 0-2 hours or over a period of time ranging from about 2 - 12 hours from its time of release.

5. A modified release dosage form of tacrolimus comprising (i) immediate release dosage unit and (ii) one or more delayed release dosage units wherein said dosage form when tested in a USP Type II apparatus using a pH change method exhibits a dissolution profile substantially corresponding to: more than 20% of the tacrolimus is released in 0.5 hours; after 4 hours 20-60% of the tacrolimus is released; and after 8 hours not less than 70% of the tacrolimus is released.

6. A modified release dosage form of tacrolimus comprising at least two dosage units wherein at least one dosage unit is an immediate release dosage unit and the at least second dosage unit is a delayed release dosage unit wherein the immediate release dosage unit comprises tacrolimus and optionally one or more excipients and the at least one delayed release dosage unit comprises tacrolimus, a delayed release material and optionally one or more excipients.

7. A modified release dosage form of tacrolimus according to claim 6 wherein the delayed release material is selected from a group consisting of enteric, water-soluble and water-insoluble materials coated on to the delayed release dosage unit.

8. A modified release dosage form of tacrolimus according to claim 6 wherein the immediate release dosage unit is a solid dispersion of tacrolimus in a water-soluble carrier coated or adsorbed on an inert material selected from beads, non-pareil seeds, granules, pellets, particles and core tablets.

9. A modified release dosage form of tacrolimus according to claim 8 wherein the water-soluble carrier is selected from the group consisting of polyethylene glycols, polyoxyethylene oxides, polaxomers, polyoxyethylene stearates, poly-epsilon caprolactone, polyglycolized glycerides, polyvinyl pyrrolidones, polyvinyl-polyvinyl acetate copolymers (PVP-PVA), polyvinyl alcohol (PVA), polymethacrylic polymers, cellulose derivatives including hydroxypropyl methylcellulose (HPMC), hydroxypropyl cellulose (HPC), methyl cellulose, sodium carboxymethyl cellulose, hydroxyethyl cellulose, pectins, cyclodextrins, galactomannans, alginates, carragenates, xanthan gums and mixtures thereof, preferably the HPMC.

10. A modified release dosage form of tacrolimus according to claim 8 wherein the weight ratio of tacrolimus to the water-soluble carrier is in the range of from about 2 : 1 to about 1 : 10.

11. A modified release dosage form of tacrolimus according to claim 6 wherein the immediate release dosage unit comprises at least one hydrophilic surfactant and at least one lipophilic additive.

12. A modified release dosage form of tacrolimus according to claim 6 wherein the delayed release dosage unit is a solid dispersion of tacrolimus in a water-soluble carrier coated or adsorbed on an inert material selected from beads, non-pareil seeds, granules, pellets, particles and core tablets which is further coated with an enteric coating material.

13. A modified release dosage form of tacrolimus according to claim 6 wherein the immediate release, the one or more delayed release dosage units or both are coated with a film-forming material selected from the group consisting of water-soluble or water-insoluble polymer(s), and natural, semi-synthetic or synthetic polysaccharides.

14. A modified release dosage form of tacrolimus according to claim 7 wherein the enteric coating material is selected from the group consisting of cellulosic polymers, acrylic acid polymers and copolymers, vinyl polymers and copolymers and enzymatically degradable polymers or a mixture thereof, maleic acid-based polymers and copolymers, polyvinyl derivatives, zein, shellac, cellulose esters, cellulose acetate phthalates and ethyl cellulose.

15. A modified release dosage form of tacrolimus according to claim 7 wherein the enteric coating material is selected from the group consisting of polyacrylamides, phthalate derivatives such as acid phthalate of carbohydrates including amylase acetate phthalate, cellulose acetate phthalate, cellulose acetate terephthalate, cellulose acetate isophthalate, other cellulose ester phthalates, cellulose ether phthalates, hydroxypropyl cellulose acetate phthalate, hydroxypropyl ethyl cellulose phthalate, hydroxypropyl methylcellulose phthalate (HPMCP), methyl cellulose phthalate, methyl cellulose acetate phthalate, polyvinyl acetate phthalate, polyvinyl acetate hydrogen phthalate, sodium cellulose acetate phthalate, starch acid phthalate; phthalate of other compounds including polyvinyl acetate phthalate (PVAP); other cellulose derivatives including hydroxypropyl methylcellulose acetate succinate (HPMCAS), carboxymethyl cellulose, cellulose acetate trimelliate, alginates; carbomers; polyacrylic acid derivatives such as acrylic acid and acrylic ester copolymers, polymethacrylic acid and esters thereof, polyacrylic methacrylic acid copolymers, methacrylic acid copolymers (for example Eudragit L and Eudragit S); styrene-maleic acid dibutyl phthalate copolymer, styene and maleic acid copolymers; shellac, starch glycolate; polacrylin; vinyl acetate and crotonic acid copolymers; poly (methyl acrylate, methyl methacrylate, methacrylic acid) and mixtures thereof.

16. A modified release dosage form of tacrolimus according to claim 7 wherein the water soluble and water-insoluble material is selected from a group consisting of ethyl cellulose, cellulose acetate, cellulose nitrate, cellulose derivatives selected from the group of hydroxypropyl methylcellulose, hydroxypropyl cellulose, methyl cellulose, sodium carboxymethyl cellulose, hydroxyethyl cellulose, poloxamers, polyethylene stearates, poly-s-caprolactone, polyvinyl pyrrolidone, polyvinylpyrrolidone-polyvinylacetate copolymer, polymethacrylic polymers and polyvinyl alcohol, polyethylene oxide and mixtures thereof.

17. A modified release dosage form of tacrolimus according to claim 6 wherein the delayed release material is in the form of a matrix and is selected from the group consisting of water-miscible polymers, water-insoluble polymers, acrylic and methacrylic acid based polymers and copolymers, hydroxypropyl methyl cellulose, polyvinyl pyrrolidone, and ethyl cellulose, oils and oily materials, gums, substituted or unsubstituted hydrocarbons, fatty acids, fatty alcohols, glyceryl esters of fatty acids, minerals, vegetable oils and waxes.

18. A modified release dosage form of tacrolimus according to claim 6 wherein the excipients are selected from the group consisting of fillers, diluents, binders, lubricants and solvents.

19. A modified release dosage form of tacrolimus according to claim 6 is a solid oral unit dosage form comprising at least one immediate release dosage unit and at least one delayed release dosage unit in the form of granules, pellets, beads or mini-tablets.

20. A modified release dosage form of tacrolimus according to claim 19 wherein the solid oral unit dosage form is a tablet, capsule or sachet.

21. A method for preparation of the modified release dosage form of tacrolimus of claim 6 comprising the steps of:
a) formulating a first amount of tacrolimus optionally with one or more excipients to form an immediate release dosage unit;
b) formulating a second amount of tacrolimus with a delayed release material to form a delayed release dosage unit;
c) optionally formulating a third amount of tacrolimus with a delayed release material to form a second delayed release dosage unit; and
d) incorporating all the dosage units together to form the dosage form of claim 1.

22. A modified release dosage form of tacrolimus according to claim 1 wherein the dosage form exhibits at least one of (a) an in-vitro release profile wherein 0- 50% of the total drug is released in 2.0 hrs; after 4 hrs 20-60% of drug is released and not less than 70% of total drug is released after 8 hrs when tested by pH change method and (b) an in-vivo plasma profile wherein the peak to trough ratio after a single dose oral administration ranges from about 6.5 to 1.5.

23. A modified release dosage form of tacrolimus according to claim 1 when administered orally once-daily provides a AUC_{0-inf} / AUC_{0-inf} of an immediate release product administered twice-daily of about 0.9 to about 3.0, preferably about 1.0 to about 2.0.

24. A modified release dosage form of tacrolimus according to claim 1 which provides a plasma concentration at 24 hours (C₂₄) substantially similar to the (C₂₄) of an immediate release product and at least about 1.3 times; at least about 1.5 times; at least about 1.8 times that of a prolonged release product.

25. A modified release dosage form of tacrolimus according to claim 1 wherein the dosage form exhibits at least one of (a) an in-vitro release profile wherein 0- 50% of the total drug is released in 2.0 hrs; after 4 hrs 20-60% of drug is released and not less than 70% of total drug is released after 8 hrs when tested by pH change method and (b) an in-vivo plasma profile wherein the steady state fluctuation (Flux) is reduced by about 40% to 70% of an immediate release product or prolonged release product.

26. A modified release dosage form of tacrolimus according to claim 6 wherein the Tₘₐₓ of first dosage unit ranges from about 0.1 to 5 hours and Tₘₐₓ of second dosage unit ranges from about 3 to 10 hours.

27. A modified release dosage form of tacrolimus according to claim 6 comprising (a) between about 0.05% w/w and about 50.0% w/w of tacrolimus; (b) between about 1.0% w/w and about 40.0% w/w of hydrophilic surfactant; (c) between about 0.1% w/w and about 30.0% w/w of lipophilic additive; and (d) between about 1.0% w/w and about 30% w/w of delayed release material of the total weight of the dosage form.

28. A modified release dosage form of tacrolimus according to claim 6 comprising (a) between about 0.05% w/w and about 50.0% w/w of tacrolimus; (b) between about 0.01% w/w and about 50.0% w/w of water-soluble carrier; and (c) between about 1.0% w/w and about 30% w/w of delayed release material of the total weight of the dosage form.

29. A modified release dosage form of tacrolimus according to claim 6 comprising (a) between about 0.05% w/w and about 50.0% w/w of tacrolimus; (b) between about 1.0% w/w and about 40.0% w/w of vitamin E TPGS; (c) between about 0.1% w/w and about 30.0% w/w of lipophilic additive selected from glycerol monooleate or propylene glycol monolaurate; (d) optionally between about 0.01% w/w and about 10.0% w/w of hydroxylpropyl methyl cellulose; (e) between about 1.0% w/w and about 30% w/w of delayed release material selected from methacrylic acid co-polymer or acrylate polymer and (f) between about 1.0% w/w and about 90.0% w/w of other excipients.

30. A modified release dosage form of tacrolimus according to claim 6 comprising (a) between about 0.05% w/w and about 50.0% w/w of tacrolimus; (b) between about 0.01% w/w and about 50.0% w/w of hydroxypropyl methyl cellulose; (c) between about 0.01% w/w and about 20.0 % w/w of surfactant selected from sodium lauryl sulfate or dioctyl sodium sulfosuccinate; (e) between about 1.0% w/w and about 30% w/w of delayed release material selected from methacrylic acid co-polymer or acrylate polymer and (f) between about 1.0% w/w and about 30.0% w/w of other excipients of the total weight of the dosage form.
